# EUROPEAN PATENT APPLICATION

(11) **EP 2 108 706 A1**
(43) Date of publication of application: **14.10.2009**
(21) Application number: 08305094.8
(22) Date of filing: 10.04.2008
(51) Int. Cl.: C12Q 1/68

(54) **New method for selecting compounds useful for treating and/or preventing microbial Shigella infection**

(71) Applicant: INSTITUT PASTEUR, 75015 Paris (FR); INSTITUT NATIONAL DE LA SANTE ET DE LA RECHERCHE MEDICALE (INSERM), 75013 Paris (FR)
(72) Inventor: Sperandio, Brice, 91760 Itteville (FR); Pedron, Thierry, 78180 Montigny le Bretoneux (FR); Sansonetti, Philippe, 75014 Paris (FR)
(74) Representative: Warcoin, Jacques

(57) **Abstract**

The present invention provides a method for the selection of compounds, which permit to enhance the level of expression of a gene implicated in antimicrobial defence comprising the step of: a) contacting a test compound with an host cell expressing a reporter nucleic acid comprising a nucleic acid sequence coding for a reporter placed under the control of a promoter, which promoter comprises all or part of the promoter sequence of said gene implicated in antimicrobial defence, and b) measuring the level of expression of the reporter gene. The present invention further relate to new compounds identified by such method, and to pharmaceutical composition comprising such compounds, and the use of such compound for preventing and/or treating an microbial infection or for preventing and/or treating cancer in a subject.

SEQUENCE LISTING

<110>  INSTITUT PASTEUR
       INSERM
       SPERANDIO, Brice
       PEDRON, Thierry
       SANSONETTI, Philippe

<120>  NEW METHOD FOR SELECTING COMPOUNDS USEFUL FOR TREATING AND/OR
       PREVENTING MICROBIAL INFECTION

<130>  67394

<160>  24

<170>  PatentIn version 3.3

<210>  1
<211>  2988
<212>  DNA
<213>  Homo sapiens

<220>
<221>  variation
<222>  (1006)..(1006)
<223>  C replaced by T

<220>
<221>  variation
<222>  (1405)..(1405)
<223>  C replaced by T

<220>
<221>  variation
<222>  (1648)..(1648)
<223>  A replaced by G

<220>
<221>  variation
<222>  (2116)..(2116)
<223>  G replaced by A

<220>
<221>  variation
<222>  (2221)..(2221)
<223>  G deleted

<220>
<221>  variation
<222>  (2883)..(2883)
<223>  T replaced by C

<400>  1
cactgtacca ccagtagcaa taaccgtatc tttaagctgg ttctttctat tgtcaccaaa      60

ccctggagct ttgactgcca caacctgaag accaaccttt agcctattca ggatgagtgt     120

agttagagct tctccatcaa tgtctccagc aattatgacc aaaggcttac agtaagcatt     180

ggcaatttca agagcagtta caatggactg gacactagaa attttctttt catgcaacag     240

aacataggca tcctggaatt cacatttctc accttttgat gtattaataa agtatggaga     300

aatatatcct cgatcaaatt ttcatgcctt caataatttc taattcatca gtcagtgttt     360

ttccatcctt tactgtgatg atgccctttc ttccaaactt tttcattgca tcagagatga     420

tgttaccaat ttctttgtct ccatttgcag aaattgtagc aacctgtgca atttcttcag 480
gtttggtcac aggtttagac tgctttttaa gttcagcaat tacagcatca acagctaaca 540
tcacacctct cttgatttcc actggattag cacctttgct aaccttctgg aagccttatt 600
tggaaataga gcataccagt acagcagcag tgatagtgcc atcccccagt ctcttcatct 660
gtgttattgg caacatcttg gacaagttta gctccaatgc ttttatattt atcctttaag 720
tcaattgact ttgcatcagt cacaccatct tttgttactt tgggacttcc ccagctatgt 780
tcaataatta ctgttcttcc ctttggcccc attgtaatgg ctacagcctc gacaaaaagt 840
ctacactttg aagcattaag gctcggacat cagcaccaaa ttttacatct ttaccatcac 900
ttcaagtgag gtgaggagcc agtagcctgg acactggtct catctggtga aagactgtgg 960
gtaatggaag catttctgtg gggtgctggc aggacatgtg catggcgagg caggtcatca 1020
gcagcaagtg agagctgcct cttactttct aaaggtgaca tagcaaatat acaaaaaaaa 1080
ataaataaat tattaattta ggtagagcac ataaaggctt tatttcatat tccatttctc 1140
tgtatgcttt cttcaccagg aagaaatagt tttagtgtca ggaatgaatg agtctgcccc 1200
tcaattccag cctgctcaac acacaaggaa acaaagccct gacaatcaga gtgactccct 1260
ggtgactaag ctccagtcct ggatgcatat ttgtttagca gttctgacag catctgaccc 1320
agccctctct ttgcataccc catcagaacc ttcttttttt ttttttcttt tgagactgag 1380
tcttgctctg tcggaagcga ctcccgtgcc tcagcctccc aaatacctgg aattataggc 1440
gtaagccatc atgcctggct aatttttgta tttttcatgg agatggggtt ttgccatgtt 1500
ggtcaaattg gtctcacact cctgacctca tgtgatccac ctgcctcagc ctcccaaact 1560
gctgggatga caggtgtaag ccaccatgct aggctcagaa atttcctttt ataaaaatgt 1620
cattaaggat cttggctgca caatatcatt accagcttcc tttaaatcca cctctggcct 1680
gccaggaatc agggttcttc agaacctgac attttaaatg aagaggtcag gcaggtcatg 1740
aggaaagcct cattgtcccc atgtctctgt cactgctgca cccctgagac atcacagaca 1800
tggacactgg ggcctgcttg tttctcaaac tgcccttaga tcgaaagagg gaggaaccag 1860
gatgaatgcc actcattttc ccaagaaagg ccctctcctg agtgcccggg atggggctct 1920
gtccattgcc tggggccgcc aattgctact ctgggttacg gaggaaggac agggtcctga 1980
gagacaccag agacctcaca cagccctgaa aacatggggc tccttcataa gtgtttccca 2040
tcaccaacag ggagaccacg tggaggcctt gcagccccac tcggtgcttc tccaccaaat 2100
cccaagggca gtgacgctga cgtctgtgga aagcagagaa agccctggct cccaaagccc 2160
tgaagtcctg tggagctgac attccctgag tgacggtgtg aatggaagga actcaagtgc 2220
gggtggtagg ccacctcctg gcccaggcct gggtgaactc tgaggggaca catgtagtca 2280
caatcccatc ctcccattct ccttctcaga ggaaggaagt gggcatccat ctgcctcatc 2340
tctctcccgt ggggaagatg gggagtttca ggggaacttt cacataaatt tcaccagctc 2400
agatctcctg tgaggatggg gcccaccatg ctcccggtgc tgccagaggc cctgagcccc 2460
tcccagggtc cctgggtttg agccagccct gtatcatccc caggagctga atgtccgagc 2520

aatggataga attagatgga aagagctctc aatttggcct gagactgtcc ccagatactc 2580

aggaaaaaca ggacgtcgca cagagtgggc agcaggtgag tggcaggtta taggtcctga 2640

gtttgagttt gttctcacgt gagacagacc cagcccctca ctccattcac acactgggtt 2700

ttaaatggtg caagatagga ggaattttct ggtcccaaga gcaggaggaa gggattttct 2760

ggggtttcct gagtccagat ttgcataaga tctcctgagt gtgcattgtt ctttgaggac 2820

cattctctga ctcaccaggt aagtggctga attctaacct ctgtaatgag cattgcaccc 2880

aataccagtt ctgaactcta cctggtgacc agggaccagg acctttataa ggtggaaggc 2940

ttgatgtcct ccccagactc agctcctggt gaagctccca gccatcag 2988

<210> 2
<211> 3504
<212> DNA
<213> Homo sapiens

<220>
<221> variation
<222> (1569)..(1569)
<223> T replaced by C

<400> 2
gaggcacatg ttgcaaaacg gtcaggtagg aagattacag acaaaaaact cattgagctg 60

gggatgtaaa aggcttggct ggacttagga agagtagttt ggacagattt cttgagatgc 120

taggatgcaa agaatttagg gacaatgggg tgatggaaaa gggtacacac ctaagtatag 180

accactacac atgagtgtag atcaccaata gtcaagggat gactctgaga gcaggcagag 240

aaatgactgg acatgctggc ttcaggtcac aatgctgcaa ttcactacag catgtcattc 300

aacctctctg agcctcagtg tcttcatcta taaaattgga aaaatgtctg cctttatttg 360

tataacttat ataatggtgg ttgtgacaag attaatggct gcaaaggaga atggctgagc 420

caaccttcat cttagactat gtcaagatga agaggagttg acaccctgga ttcccgtgct 480

gggagcccat gccagagtct tgatattgtt aaccgagggt cacgtgggtg atacttgggt 540

gatacatggg tcacatggtg atttgcaatg tacaagtcaa tttcacacca ttaactcata 600

gggctttcac tgtgacctaa acaggaaatg tactgctctc tgtgctttgt tgctgagtta 660

cagatgctca cagagcttag gtaatttatt caggtaaacc cacgccatac acggtagggc 720

tgagtgatgg cccaatgacg cctcacttca gacctggtgt ttgtcttcta catagctctg 780

cccttctgcc acaaacttgg accaatcaca tctcagctcc acatattaag aacaggattt 840

aaaaaataat aattaaagga aagtctgaca tgaacaaagg tgataaataa ctaaagtttc 900

tcttccttgc tcaacctaat caaaatgtca aaatgctggc tgtgagagct acaaggaaac 960

caagcacatg ggtacaaaaa tttgcaggat ctgcagttcc aagcgctgtg actcagagcc 1020

caggggcagg gagtgacaag acccagctgt cgttagtgtt gtatggcttc agacacatga 1080

atctattcac ccaactatta gacagcacac gtgtacttgt tatcatttga gaggctaaaa 1140

gtattgaaga cataaaaagg attataataa gccaaaaaga ggacaattca cagttgtcag 1200

tggctccctg aagtagagaa gtaaattaca cagtctctct cttctattgt ttgaaatttt 1260
accataaaaa tgcattactt ttataatttt cttaaaacag gtaagtcccc ctcaaaacgg 1320
attttgtgtg tctggagtat gtccaaactt gaaaaggctg attgtcacag cccaatctcc 1380
aattcccagc tcccaaacta gttgacaaga aaaagtggca tttttcctca ccaaggaact 1440
tttatgaaat agtggtgttg ccataaaaat gcctcccagt ctatgaagga acaggttaga 1500
ccaggggttc acctgtggaa gtctattctc catgtggaaa agcaagtcat ctccagcgtc 1560
tgactgtctt caccccaggc tgagagctgg tgctaaccct ctacaacgcc caatgccatc 1620
gttatgtctg acatagtaag actgtgaagt cagcacccat cccacccaca ctcacctcct 1680
tcccccattt ataaatatct ccaattgctt tgtctcaaat catgaactaa agaggaaagt 1740
tagattttaa aaagtagcat ctacctatct aaagtcttaa atctgctgat tgagctccac 1800
tcttggctca aagagttatc tttgagttat gatgagttat ctttcttcta cgtttctgca 1860
gccctgtgct tgaaatcttc ctatgactcc cattataatt catcctaaat ttatcatttg 1920
actgtctgcc catctacctg gccctacatg gagcttctgg agagaaagaa ccaagactgg 1980
tccctcttgg tatctcatcc acttcaccct cacctatgct gaagacatga tacttgtccc 2040
ttcctatagt atgtgttcag agttttctta ctgaatctga caggtgtggt gcacccaaat 2100
tctgaacaca cactcccaga actaacacac ccttgatcct tagtggatcc gttataattg 2160
cattcacacg aagcagcagc taggagacat cacggtgact tcagctccca attgaattgt 2220
cattgactta cttagttccc aattgatagt caaccaaata caacccatcc attggattcc 2280
tgaacattta agtttaatag ctgttttttt tttctttttt agcatttttc cctctaaacg 2340
ttgaatctat atgcagggga aatcatctgg cccctggact gccttgaatg ctcctgagcc 2400
aagagtaact tgaggtctta aatgtctatt ctgaagcccc tcttttgcac agggacagtc 2460
attgactagg aggagatgcc agtctattaa atctatcatc ccagcatcct ctttctctga 2520
tgaagtaaaa ttaaacacct tctttactca taaatttggt aggttttaga caatgatgaa 2580
gaatcggaca tgggccgcac acagtggctc ataccagtaa tcccagcaat ttaggaggct 2640
gaagcaggag gattgcttga gcccaggagt tcacatctgg gctcgaaacc agtctgggca 2700
gcatagtgag accccatttt attttctaca gaaaatttaa aatttagcca tgtgtggagg 2760
catgcgtttg tggtcccagc tactcagggg gctgaagtgg gaggacctct tgagcctgac 2820
agctcaagct gttgtgagct gtaatcatgc cactgcactc cagcctgcac aaaaaagtga 2880
gactctgtct ctcaaaaaaa aaaaaaaaaa tgaaagaatg agacatgttt cactcaaatt 2940
atagcacccc atcataggcc aagggccagg tggtcagcta accttgaatg tagcccattt 3000
tcctccctta cctcactacc aaaaagccag tcctattgcc agatattgaa tatgtccagg 3060
gctggattta tttggtcccc aggagggtct tagactctcc cagcatttta ggaagagaaa 3120
aaaagctcca aagcagcaaa gtacggacaa gtcagccccc agccacatgc cctgagacta 3180
ttccccaaag gggcatactt tgctcatgcc agccctatct gtacccacaa cccaatctga 3240

cccacaccca gacaagcccc tggagtccag agagcacccg gacagccaca aatccatagg 3300

gagctctgcc ttaccattgg gttcctaatt aactgagtga gtgggtgtgt tctgcatggt 3360

gagaggcatt ggaatgatgc atcagaaaac atgtcataat gtcatcactg taatatgaca 3420

agaattgcag ctgtggctgg aacctttata aagtgaccaa gcacaccttt tcatccagtc 3480

tcagcgtggg gtgaagccta gcag 3504

<210> 3
<211> 3445
<212> DNA
<213> Homo sapiens

<400> 3
gagtgggaga gggtagctcc agcatctgct catctgtgga aagtctctca gagccttcag 60

cagtcccagt gcaccaggtt ccctgatcac attgtgtagg agttggagcc tcctgggtct 120

gcaggagagg aaggggccac ttagagaagt cagggtggga gattcagaga gagagaggaa 180

agagagagag atagagagac agagggacag gggctgccca ggcctttgtg aagggacatt 240

tatctagccc tcaagtcttg atgtggaaaa gcctaacttt tttttttttt ttgagacgga 300

gtcttacact cttgcccagg ctggagtgca gtggcgccat ctcagctcac tgcaagctcc 360

gcctcctggg ttcacaccat tctcctgcct cagcctccca agtagctggg actacaggcg 420

catgccacca cacccagcta gagacggggt ttcactgtgt tagccaggat ggtctcaatc 480

tcctgacctc atgatcaggc cacctcggcc ttccaaagtg ctgggattac gggcgtgagc 540

caccgcgccc ggctaagcct cacttttctt ttctcattcc gccttgccta gaatcttctg 600

atcactctat taatggggtg agccattatg aaaaaggcga gctggtgaga gcctcctttc 660

tccctctcta ggaggaaggg gcagccatgg gttctaaagg tctgaagtca gccgccctac 720

ctcaggtagt ggacaccgac ctgcatgtgc cgagggcagg actcactcct ggaacctggg 780

aagcctggac cttgtgcgat ggccctgaga gggaaagcaa gtgcccctga gtccagactt 840

ctgcagttgt atcagagggc ctatgcaagc tgcttgggct ctggctgatt tcatgtgagg 900

gcgtgtccag cactggccga agtggaacgt cctcttgagg cagttggaaa ggggctctga 960

atatgccaga tctgacctag aggaacacag gagcatgcag ctctcacgcc ctagcctaag 1020

gactaacatt gccatctgct tctccccggt ttggcttctt ggggatcctg aagccagtca 1080

gaagggtgga ggaagcagga gagccctgag aggtctgata tccctggatg gcccttttcc 1140

caacagccat aaaagacctg atgtgcaagc cctgcttgac caacaggcca ctggggcaac 1200

acttgaactg tgaaaccagg ttgcacagaa gcccacttct tccttctctc aaggccaggt 1260

gtgcaaccgg tggccttcgt ccaggcttcc accttccagg agccctgctg tccaagcccc 1320

caagtgtccc acaggccaac tgaggtcaag aggtacccca ggcctcaggg ccacatggcc 1380

agtccacaga ggagctggga ccggaatttg cctcatggac gctggacccc tctatcactt 1440

tggcccttta tctctcctgg ctgtgatctc tccctctccc aacaccagcc acccatccca 1500

gcttggtgtc tcagctagag caccaaacag ggagtaattt gaggtcgaat caggagagac 1560

ttcctggaag atgtaggggc aaagaggaga ttgctgtgcc tggttggagg tcagggaaag 1620
cttcatgagg tgaaagggca ggccaaacag aaggatcggg gcagagctgg gagaaagagc 1680
aggaggctca gatgagccta tgtgatgggg gcatacgtga cagtctggca cttgcagtgt 1740
gcagggctgg ggtgagggca gtgggaacca aaaggccaca ggccctgatg ccagcctagg 1800
gggtgtgtgg gctcctgaag gtagagggtt gaaatggcca aattggtggt ttaaaggaac 1860
ccctggacaa cggtgtggag ggtggactgt ggtctgggga gagcctgaaa cctagagaag 1920
gtggagtctg gaggctgggg cagcattgca tgggagagaa gatggcggca ggagccaggc 1980
aggggtggaa ggagaggcca gaggggagtg ctttcgggag gaaggagcca aaggtgcttc 2040
agggctcatg cagtgggcac tcagcagaag agcagtcaag gaggcccagt ggggacaacc 2100
gggacgttca tgggagtctc gaactcagag atacagactg ggtggtgtct gcctattata 2160
actggtgtcc tgggagaggg cgtccccagg gaaacccgag gccagggaag agaagcagcc 2220
caggaggtga ccccacacgc tctcaatatt aagaagacag cccttagggg agaccaagaa 2280
ggagccacca gggaggcagg agggacagca ggagagtggc atcaggggag accggggcag 2340
caagaaggaa ggctcaccag tgcctgtgca gacaaagacc aggcttgagg gtggccatgg 2400
accaagggtg ggttggccag ccttcagtgg ccttcagcat gaagaagagt gaaaagatgg 2460
ggtcaagaag cgtagacaac ctttcctaag acttggcttg ggagaagcca tggtgaaagg 2520
ccatgtctgg aggggcttgg gaacattttg agactgaaaa aaacagtgaa gaaggatgca 2580
gaaggggata gatggagcag agccttcgtc tggctgacgg ctgggtccag agagcatgtg 2640
gtatggcctt gaactgaaag ggcaacttgt cccttgcaag agtgagtctc taggttgggg 2700
gtggctactg tcttcattta ccagttcttt tttttttttt tcatactgag tctcactctg 2760
ttacccaggc tggagtgcag tggcatgatc tcagctaact gcaacttctg cttcccgggt 2820
tcaatgggtt caagtgattc tcatgcctca gcttgtagct gggactacag gtgtgagcca 2880
tcatgcgtgg ctaattttca tatttttagt agagatgggg tttcaccatg ttggccaagc 2940
ttgtctcgaa ctccttatct caggtgatcc gcccaccttg gcctcccaaa gtgctgggat 3000
tataggcgtg agccaccgtg ccctgcctca ttcatcaatt cttaatcgat gcctacaggg 3060
tgccaggcaa tgcctagagc tggagattta gcagtccatc atactgactc ctgaggagta 3120
gaaggatgta gaataggcac ctggctctct tcctctctgg agggatttaa cgctcttgag 3180
cacccctggc tatgacaatc tccggtcagg tctgggaggt tgtcagagat gaagaaacca 3240
cttcctcatc ttgcacacaa ggaaggcctc actcactgcc cagcaagtcc tgtgaagcaa 3300
tagccagggg ctaaagcaaa ccccagccca caccctggca ggcagccagg gatgggtgga 3360
tcaggaaggc tcctggttgg gcttttgcat caggctcagg ctgggcataa aggaggctcc 3420
tgtgggctag agggaggcag acatg 3445

<210> 4
<211> 4000
<212> DNA
<213> Homo sapiens

<400> 4
tctacaaaga agccagccag gattttaata gaaattgtgt taaataggcc gggcatggtg 60
gctcacacct gtaatctcaa cactttagga gtctgaggtg ggcggagcac ctgaggtcag 120
gagttcaaga ccagactggc caacatggcg acaccctgtc tctactaaaa atacaaaatt 180
agcaaggtgt gatggtgcat gattgtagtt ccaactactt gggaggctga ggcaggagaa 240
ttggctgaac ctgggaggca gaggttgcag tgagccgaga ttgcgccatt gcactccagc 300
ccgggcaaca agagtgaaac tccatctcaa aaaagagaga gagagagagg aaaagaaaag 360
aaattgtgtt gaatagaagc ggtgagactg aacatccttg tcttgtcctt gatctcaggg 420
aaaaggcagt aagtctttca acattgagtg tgctatcagc tatgaggcag tcatagatga 480
cctttatcag gttgagatgt tctctgctat tcctagtttg ttgagtgttt tgtcatgaga 540
aggtgctgga ttttgttagt tacttttcat atagttttta tttttattca attaatttgt 600
agtattacat ttcctggaat aaatcccact tgttatggtg tatggtcctt ttactatgtt 660
gttttgtttt tgtgtttgta ttcggaaagg acattggtct gttgttttct tttcttgtga 720
tgtgtttatc ttattttggt gtcaggataa tactgtcctc atagaagaag ttgaagaggg 780
ttcccttctc ttctatactt tgaaaaagtt tgtgaagaac tggtattaat tcttatttga 840
atgttttgta gaattaacca gtgaattaat ctggtcctga ggttttattt gtgaaaaata 900
tttggttatc aatttgatat ctttacttgt tatagctcta ttcaaatttt ctattttttt 960
tttgagatgg agtcttgctg tgtcgcccag gctggagtac aatgacacga tcttggttca 1020
ctgcaacctc tacctcccgg gttcacgcca ttctcctgcc tcagcctccc tagtagctgg 1080
gatcacaggt gcccgctacc acaccaggct aattttttgc atttttagta gagacagggt 1140
ttcaccatgt tagccaggat ggtctcgatc tcctgacctt gtgctccttc cgccttggcc 1200
tcccaaagtg ctgggattac aggcatgagc caccacgccc atcccaaatt ttctattttc 1260
ttcttgaatc agggtcaata atttgtgtct ttcatctcta ttcgtatcag ctgtgaggtg 1320
gtcatagatg acttttatca ggttgagaaa gttctcaact attcctagtt agttgagtgt 1380
ttttgtcatg acaacgtgtt ggattttgtc agttgctttt caaatcattt ttatttttta 1440
tttgattgat ttgttgtatt acatttcctg gaataaatcc cacttggtta tggtgtatga 1500
tctttttact gtgctgtcgg catagtaatt ttatttgcat acagtttttt tttttttttt 1560
ttttttttta cctccctttc ctttgtttgg tttggtctag tcaaggcttg ttagttttgt 1620
tgatcttctc aaagaactaa ctttcgcatt cgttgatttc ctctattgtt ttgcattctc 1680
tgttttattt attcctgctc taatcttcat ttccctcctt ctgcatgctt tgggtttagc 1740
ttgctctgct tttctctagt ttcttaaggt agaagtttgg gttattgttt tgagatgttt 1800
cttttttttt ttttttaatg tgagtgttta cagctataag tttccttctg aggactgctt 1860
ttgctacatc ctataagttt tgatatgttg tggacttttt tttacatttc aaagttgttt 1920
tctactttct ctagtgactt ttcttttgac acattgttgt tgttgttgtt gttttttcct 1980
ttgagatgga gtctctgttg gccaggttag agggcagtag cgtgatctcg gctcgccgta 2040

acttctgctt cctgggctca tgcgattctc ctgcctcagc ctcccaagta gctgggatta 2100
caggcatgga ctaccacacc cggctaatat ttgtattttt agtagagaca gggtttcact 2160
atgttggcca gtctggtctc gaactcctga cctcaggcag tccacccacc tcggcctccc 2220
aaaatgctag gattacaggc atgagccacc actcctggcc agacacattg gttatttagg 2280
aatatgtggt ttcctttctg tctgttactg atttctgatt taattccatt gtggtttgaa 2340
tgtatacttt atgtgatttc agtcggttta catttactga gacttgttcc ataacctacc 2400
atatgcttta tcctagagaa cattccatgt gcaactattc tgttgatgtt ggatagagta 2460
gtccttcgat gcctgctaag tctatttggc ttatagtgtt gttcaagtct tctattttct 2520
tgtggatctt ctgcctaaat tttattaatt agtgaaaatg tggtaccaaa gctgtcaact 2580
tttattgtta aatttcctat tctgctgggc acggtggctc atgcctgtaa tgaggtcagg 2640
agttcagcct gggcaacatg gtgaaaccac gtctctacta aaaatacaaa aattcgctgg 2700
gcgtggtggc acgtgcctgt agtcccagct actcaggagg ctgaggcagg cgaatcgctt 2760
gaacccagga ggcggaggtt gtagtgagcc gagatggtgc cactgcactc cagcctgggc 2820
aacaagagca aaattctgtc tcaaaagaaa ttataataat aaaataaaat aaaatttcct 2880
attcttccct tcaattctgt cagtttttgc tttctgtagt ttggggtgct actgttaggt 2940
atatatatgg ttataggttt tacatcttcc caatgggtta attttgtgtt accatgaaag 3000
tcccttttta tttccggtaa cattaaaaaa aaatctattt tgtctgatat aggcatcacc 3060
aactccagtt ctcttttgtt tactgtttgg tatacctttc ttttgatcct tttactttca 3120
acctatttgg gtctagtgga gagttcttat actgccttag gttagggatt catattacat 3180
tatcatcatt agtactgtta tttgacattt gctgtgctga ctagctactg ctgataggtt 3240
ttctttcctc aacaattctg aggctctata ttgagttata ttagtacatc atcatggaga 3300
gttaaaggta ggtaaggatt attttctgaa ctgcaatatt gattaaagcc atgtgaatgt 3360
ataagattct tagaagagtt gacattaaat caaggtgaag ctgaggtttg agccttactt 3420
aaaggctgat attttccact ctaactgcgg acagtactgt agcactgtta tagtacctgc 3480
tctgaatgtt agtctagcaa ctcagggtct tcttcatgac agctgaacct caaccatgtg 3540
atggtaaatg tgtagcagag tatgcctggc atcccacctg ctcctcctcc ccctcctcct 3600
tgactggttc tggaaagcaa atagggtgta acaataggag ttctggaatg ttcctgtgtg 3660
gggctgacct ttgtatcgct gttaatcctc tattttcaga cacaaaaatg attaagttaa 3720
aactggatga aagtcttttc tgggtcacag ggctgagctg cttttgctct ttgcaaatac 3780
aaagaattta acaggattct ccccttctca acttcctgtc ccccaccctg accttcgcac 3840
cttcccaata tgaggaaaaa gcaggaagtt ttccttgcgg gtttttttta tgatgacatg 3900
atggggccag ttgatcaatg gggaaaaccc catgtggcaa cacgccttct gtgtacattc 3960
ccaatatttg ctataaatag ggccatccca ggctgctgtc 4000

<210> 5

<211>  22
<212>  DNA
<213>  Artificial

<220>
<223>  primer

<400>  5
caggtggtaa ctttctcaca gg                                                22


<210>  6
<211>  22
<212>  DNA
<213>  Artificial

<220>
<223>  primer

<400>  6
aatagagaca ttgccctcca ct                                                22


<210>  7
<211>  21
<212>  DNA
<213>  Artificial

<220>
<223>  primer

<400>  7
gccatgaggg tcttgtatct c                                                 21


<210>  8
<211>  21
<212>  DNA
<213>  Artificial

<220>
<223>  primer

<400>  8
ttaaggcagg taacaggatc g                                                 21


<210>  9
<211>  21
<212>  DNA
<213>  Artificial

<220>
<223>  primer

<400>  9
gtgtgttctg catggtgaga g                                                 21


<210>  10
<211>  21
<212>  DNA
<213>  Artificial

<220>
<223>  primer

<400>  10
tataaaggtt ccagccacag c                                                 21

<210>  11
<211>  23
<212>  DNA
<213>  Artificial

<220>
<223>  primer

<400>  11
cggaaatcta aagagaagat tgg                                              23


<210>  12
<211>  22
<212>  DNA
<213>  Artificial

<220>
<223>  primer

<400>  12
tagggcacac actaggactc tg                                               22


<210>  13
<211>  22
<212>  DNA
<213>  Artificial

<220>
<223>  primer

<400>  13
gacatagccc aagaacagaa ag                                               22


<210>  14
<211>  22
<212>  DNA
<213>  Artificial

<220>
<223>  primer

<400>  14
taattggaca agtccagtga gg                                               22


<210>  15
<211>  22
<212>  DNA
<213>  Artificial

<220>
<223>  primer

<400>  15
cagtcatcat ctccagctca ac                                               22


<210>  16
<211>  22
<212>  DNA
<213>  Artificial

<220>
<223>  primer

<400>  16
ctccgagaca gtctggtact tg                                                22

<210>  17
<211>  26
<212>  DNA
<213>  Artificial

<220>
<223>  primer

<400>  17
cactgtacca ccagtagcaa taaccg                                            26

<210>  18
<211>  26
<212>  DNA
<213>  Artificial

<220>
<223>  primer

<400>  18
ctgatggctg ggagcttcac caggag                                            26

<210>  19
<211>  26
<212>  DNA
<213>  Artificial

<220>
<223>  primer

<400>  19
gaggcacatg ttgcaaaacg gtcagg                                            26

<210>  20
<211>  26
<212>  DNA
<213>  Artificial

<220>
<223>  primer

<400>  20
ctgctaggct tcaccccacg ctgaga                                            26

<210>  21
<211>  26
<212>  DNA
<213>  Artificial

<220>
<223>  primer

<400>  21
gagtgggaga gggtagctcc agcatc                                            26

<210>  22
<211>  26
<212>  DNA
<213>  Artificial

<220>
<223>  primer

<400>  22
catgtctgcc tccctctagc ccacag                                          26


<210>  23
<211>  26
<212>  DNA
<213>  Artificial

<220>
<223>  primer

<400>  23
tctacaaaga agccagccag gatttt                                          26


<210>  24
<211>  26
<212>  DNA
<213>  Artificial

<220>
<223>  primer

<400>  24
gacagcagcc tgggatggcc ctattt                                          26

## Description

The present invention relates to a new method for selecting compounds which can be useful for treating and/or preventing microbial infection, to new compounds identified by such method, and to pharmaceutical composition comprising such compounds.

The efficiency of antimicrobial host defence in the human intestinal tract relies on the capacity of the mucosal immune system to recognize and neutralize pathogens. In this context of innate immunity, sensing of the bacteria occurs mainly through engagement of host cell Toll-like receptors (TLRs) and other pattern-recognition molecules, such as the nucleotide oligomerization domain proteins (NOD), by pathogen-associated molecular patterns (PAMPs)(FRITZ et al.. Nature immunology, vol.7, p:1250-1257, 2006; HOFFMANN & KAFATOS, Science, vol.284, p:1313-1318, 1999 ; JANEWAY & MEDZHITOV, Annual review of immunology, vol.20, p:197-216, 2006). Following the pathogen recognition step, the subsequent antimicrobial response is achieved by recruitment of immune cells and synthesis of antimicrobial factors by the injured mucosa, including production of molecules such as defensins, cathelicidins, lysozyme, phospholipases and proteases that exhibit a broad range of actions against a variety of pathogens (SELSTED & OUELLETTE,. Nature immunology, vol.6, p:551-557, 2005; ZASLOFF, Nature, vol.415, p:389-395, 2002).

Among enteric pathogens, virulent *Shigella* are highly contagious Gram-negative enteroinvasive bacteria that cause bacillary dysentery. In malnourished toddlers in the developing world, untreated infections can be fatal. *Shigella* is unable to invade intestinal epithelial cells through the apical surface but needs to translocate the intestinal epithelium for the development of infection (MOUNIER et al., Infection and immunity, vol.60, p:237-248, 1992). The pathogen translocates through M cells of the follicle-associated epithelium that covers the lymphoid nodules associated with the colonic mucosa. In the subepithelial location, *Shigella* causes apoptosis of resident macrophages, allowing bacterial escape into the tissues and efficient basolateral entry into epithelial cells, followed by cell-to-cell spread and efficient intracellular growth *(*WASSEF et al., Infection and immunity, vol.57, p:858-863, 1989; ZYCHLINSKY et al., Nature, vol.358, p:167-169, 1992). Subsequent inflammation destroys cohesion of the epithelial barrier, facilitating further invasion of luminal bacteria and propagation of the infection (PERDOMO et al., The Journal of experimental medicine, vol.180, p:1307-1319, 1994).

Recent studies reported that *Shigella* may interfere with this host barrier defence system, which immune escape strategy may also be used by other human pathogens.

In the present application, by combining *in vitro* and *in vivo* approaches, the inventors now demonstrate that *Shigella flexneri* actively dampens host innate immune responses by suppressing transcription of specific antimicrobial peptide genes. The inventors identified bacterial effectors controlled by the MxiE transcriptional activator as the molecules responsible for this mechanism. *In vivo,* the strategy employed by *Shigella* to modulate transcription of these target genes was followed by a decrease in the production of these epithelial cell encoded peptides, coupled to a deeper progression of denser population of luminal bacteria towards the crypts. The inventors demonstrate that MxiE-dependent effectors are also able to downregulate additional innate immunity genes, such as chemokine genes (e.g., CCL20), leading to a negative regulation of the DCs recruitment to the lamina propria of infected human tissues. The production of antimicrobial molecules and recruitment of immune cells is one of the major immune responses dedicated to the protection of the mucosal surfaces. Thus, this work highlights the strategy developed by *Shigella* to subvert the dynamic defence processes of the intestinal epithelium and has enabled to develop a method for selecting compounds with the capacity to thwart this strategy.

The present invention provides a method for the selection of compounds, which permit to enhance the level of expression of a gene implicated in antimicrobial defence comprising the step of:
a) contacting a test compound with an host cell expressing a reporter nucleic acid comprising a nucleic acid sequence coding for a reporter placed under the control of a promoter, which promoter comprises all or part of the promoter sequence of said gene implicated in antimicrobial defence, and
b) measuring the level of expression of the reporter.

By "compound" or "test compound", one should understand compounds of different nature, structure and origin, particularly biological compounds, nuclear factors, cofactors, and the like, chemical, synthetic compounds and the like, which are tested for their capacity of enhancing the level of expression of said gene implicated in antimicrobial defence.

The concentration of said test compound can be adjusted by the skilled person according to the characteristics of said compound (its toxicity, ability to penetrate cells, etc.), the number of cells, the length of the incubation period, etc. Generally, the cells are exposed to concentrations of test compounds ranging from 1 nM to 1 mM. Of course it is possible to test other concentrations without deviating from the invention, and also to test simultaneously different test compound concentrations.

Different adjuvants and/or vectors and/or products facilitating the penetration of the test compounds into the host cell such as liposomes, cationic lipids or polymers can also be used, when necessary.

By "enhance the level of expression of a gene implicated in antimicrobial defence", one should understand that the expression level of a gene implicated in antimicrobial defence is improved compared to a control level. As an example, the level of expression of a gene implicated in antimicrobial defence is considered to be improved, when its level of expression after induction by the test compound is greater (e.g., at least two fold) than its basal level. As another example, the level of expression of a gene implicated in antimicrobial defence is improved, when its expression is induced by the test compound whereas this gene is not expressed at its basal level.

It should be noticed that said expression level of a gene implicated in antimicrobial defence is correlated to the expression level of the reporter gene in the method of the invention, which reporter gene is placed under the control of a promoter comprising all or part of the promoter sequence of said gene implicated in antimicrobial defence. In fact, one of skilled in the art can deduce that a test compound can enhance the expression level of the gene implicated in antimicrobial defence from the capacity of said compound to obtain an enhanced expression level of the reporter gene in the method of the invention.

In the present invention, the control level can be determined, by example, by measuring the expression level of the reporter gene in the absence of the test compound.

Thus, in a preferred embodiment, the method according to the invention further comprises a step c) of comparing the level of expression of the reporter gene as measured in step b) with the level of expression of the reporter gene in the absence of said test compound.

As used herein, the expression "a gene implicated in antimicrobial defence" should be understood as a gene coding for an antimicrobial factor and/or a chemotactic factor for immune cells.

Preferably, the gene implicated in antimicrobial defence codes for an antimicrobial factor and/or a chemotactic factor for immune cells, which is active against Gram-positive or Gram-negative bacteria infection, more preferably against *Shigella,* and even more preferably against *Shigella flexneri.*

More preferably, the gene implicated in antimicrobial defence is selected in the group comprising beta-defensins, cathelicidins, chemokins and chemokin receptors.

As an example of beta-defensins, one can cite DEFB4 (hBD2; Accession number NC_000008.9, nucleotide 7789609 to 7791647) and DEFB103 (hBD-3; Accession number AC_000140.1, nucleotide 6934691 to 6936070).

As an example of cathelicidins, one can cite CAMP (LL37, Accession number NC_000003.10, nucleotide 48239866 to 48241979).

As an example of chemokins, one can cite CCL20 gene (Accession number NC_007869.1, nucleotide 91699160 to 91702333).

As an example of chemokin receptors, one can cite CCR6 gene (Accession number NC_000006.10, nucleotide 167445285 to 167472619).

In a preferred embodiment, the gene implicated in antimicrobial defence is selected in the group comprising DEFB4 (hBD2; Accession number NC_000008.9, nucleotide 7789609 to 7791647), DEFB103 (hBD-3; Accession number AC_000140.1, nucleotide 6934691 to 6936070), CAMP (LL37, Accession number NC_000003.10, nucleotide 48239866 to 48241979), CCL20 gene (Accession number NC_007869.1, nucleotide 91699160 to 91702333), and CCR6 gene (Accession number NC_000006.10, nucleotide 167445285 to 167472619).

Preferably, said gene implicated in antimicrobial defence is selected in the group consisting of DEFB4 (hBD2; Accession number NC_000008.9, nucleotide 7789609 to 7791647), DEFB103 (hBD-3; Accession number AC_000140.1, nucleotide 6934691 to 6936070), CAMP (LL37, Accession number NC_000003.10, nucleotide 48239866 to 48241979), and CCL20 gene (Accession number NC_007869.1, nucleotide 91699160 to 91702333).

In a preferred embodiment, the selected compound does not enhance the level of expression of a gene implicated in inflammatory response, preferably said compounds does not induce inflammation.

As used herein, the expression "does not enhance the level of expression of a gene" means that the expression of said gene is not induced by the test compound or that the expression of said gene is induced from less than two fold by this test compound compared to the basal level of expression from this gene.

Preferably, the method of the invention further include the step d) of measuring the level of expression of at least one gene implicated in inflammatory response.

Optionally, this step d) can be followed by a further step of comparing the level of expression of the gene implicated in inflammatory response in absence of the test compound.

Genes implicated in inflammatory response are well known from the skilled person and include, as an example, ICAM-1, TNF-α and IL-8.

Again advantageously, the selected compound does not enhance the level of expression of a gene implicated in inflammatory response, but enhance the level of expression of a gene implicated in epithelial cell turnover.

The step of "contacting" the test compound with the host cell can be achieved on any suitable support and in particular on a plate, dish, in a tube or flask. Generally, said contacting step is carried out on a multiwell plate which allows many different assays to be carried out at the same time. Typical supports include microtitration plates and more particularly plates with 96 or 384 wells (or more), which are easy to manipulate.

The protocol of said contacting step may depend on the nature of the test compound, on its concentration, on the nature of the host cell, and on other technical considerations.

Said contacting step is typically maintained for several minutes to several hours, generally between 1 and 48 hours. In particular, the host cell and test compound should preferably remain in contact long enough to allow de novo synthesis of the reporter gene expression product.

The term "a reporter" means any polypeptide known to those skilled in the art whose activity or presence in biological extracts is easily measurable, and which can be used as reporter to carry out the screening method.

For example, said reporter can be luciferase, and more particularly firefly or Renilla luciferase, secreted alkaline phosphatase, galactosidase, lactamase, chloramphenicol acetyl transferase (CAT), β-glucuronidase (Gluc) and green fluorescent protein (GFP). Preferably, said reporter corresponds to green fluorescent protein (GFP) and more particularly to enhanced green fluorescent protein (eGFP).

The nucleic acid sequence coding for the reporter is comprised in a reporter nucleic acid, wherein said nucleic acid sequence, whatever it may be, is placed under the control of a promoter containing all or part of the promoter sequence of the gene implicated in antimicrobial defence.

Said part of said promoter sequence advantageously comprises at least 10 consecutive nucleotides of said sequence, preferably at least 20, more preferably at least 30, even more preferably at least 50 consecutive nucleotides. The promoter can also comprise heterologous regions, originating from other genes, such as for example silencer or enhancer sequences conferring regulated or tissue-specific features, etc. Said particular sequences can be present in one or more copies in the promoter (preferably 1 to 10 and more preferably 1 to 6), upstream, downstream, or internally to the promoter, in the same orientation or in the opposite orientation. Finally, the promoter can be a hybrid promoter combining regions from other promoters sequences, for example promoter sequences of the herpes virus thymidine kinase (TK) gene, the CMV early promoter, the PGK promoter, the SV40 promoter, etc.

Advantageously, the reporter nucleic acid comprises the promoter sequence of the gene implicated in antimicrobial defence, preferably said promoter sequence is selected in the group comprising beta-defensins promoters, cathelicidins promoters, chemokins promoters and chemokin receptors promoters. Preferably, said promoter sequence is selected from the group comprising DEFB4 promoter sequence, DEFB103 promoter sequence, CAMP promoter sequence, CCL20 promoter sequence and CCR6 promoter sequence.

More preferably the promoter sequence is selected from the group comprising human DEFB4 promoter sequence (SEQ ID NO: 1), human DEFB103 promoter sequence (SEQ ID NO: 2), human CAMP promoter sequence (SEQ ID NO: 3), human CCL20 promoter sequence (SEQ ID NO: 4), and human CCR6 promoter sequence.

Furthermore, the different aforementioned functional domains (i.e., the promoter and the reporter gene) can directly flank each other, or be separated by nucleotides which do not significantly affect the functionality of the reporter nucleic acid or which give the system improved performance or characteristics (amplifier, silencer, intron, splicing site, etc.).

Advantageously, the host cell has been transformed with a vector.

Any suitable vector can be used in order to facilitate penetration of the reporter nucleic acid in the host cell. As an example of such vectors, one can cites plasmids, cosmids, phages, or virus. Preferably, said vector is a virus, and more particularly a lentivirus.

Preferably, said vector is selected in the group consisting of plasmids pLenti/R4R2/V5DEST-pBS5 comprising human DEFB4 promoter fused to the eGFP reporter nucleic acid sequence, pLenti/R4R2/V5DEST-pBS7 comprising human DEFB103 promoter fused to the eGFP reporter nucleic acid sequence, and pLenti/R4R2/V5DEST-pBS9 comprising human CAMP(LL37) promoter fused to the eGFP reporter nucleic acid sequence, which plasmids are present in the Stbl3 *E. coli* bacterial strains deposited at the CNCM on April 4, 2008 with the numbers I-3965, I-3966 and I-3967 respectively.

More preferably, said vector is selected in the group consisting of lentivirus obtained by recombination of the abovementioned plasmids pLenti/R4R2/V5DEST-pBS5, pLenti/R4R2/V5DEST-pBS7 and pLenti/R4R2/V5DEST-pBS9.

Said recombination reaction is done according to the manufacturer's instruction of the VIRAPOWERTM PROMOTERLESS LENTIVIRAL GATEWAY®) kit (INVITROGEN) disclosed hereafter in the examples.

The construct or vector can be introduced into a host cell by any conventional method, including in particular electroporation, calcium phosphate precipitation, liposomes, transfectants, infection, etc.

The host cells which are used can be selected from among any cells that can be cultured in the laboratory.

In another preferred embodiment, the host cell is a mammalian cell (e.g., hepatocytes, fibroblasts, or epithelial cells, etc.). Even more preferably, said host cell is of human origin. Said host cell can be obtained from primary cultures or established cell lines.

Preferably, said host cell is infected with *Shigella,* preferably *Shigella flexneri.* This infection can be achieved as described in the examples.

In a specific embodiment, said host cell is an epithelial cell, preferably a colonic epithelial cell, and more preferably human colonic epithelial cells HT29 (ATCC HTB-38D), Caco-2 (ATCC HTB-37) or TC7 cell line which is a sub clone of Caco-2 disclosed in CHANTRET et al. (J. Cell Sci., vol.107, p:213-225, 1994).

Advantageously, said host cell is selected in the group comprising TC7 human colonic epithelial cells expressing the eGFP gene under the control of DEFB4, DEFB103, or CAMP promoter and HT29 human colonic epithelial cells expressing the eGFP gene under the control of DEFB4, DEFB103, or CAMP promoter.

In another specific embodiment, said host cell is a leucocyte, preferably a macrophage, and more preferably a U937 cell (ATCC CRL-1593.2).

In another alternative embodiment, it is also possible to use prokaryotic cells (bacteria), yeast cells (*Saccharomyces, Kluyveromyces,* etc.), plant cells, etc.

Depending on the support and the nature of the test compound, variable amounts of host cell can be used to carry out the aforementioned methods. Classically, between 10³ and 10⁶ host cells are contacted with a particular test compound, in a suitable culture medium, and preferably between 10⁴ and 10⁵ host cells. As an example, in a 96-well plate, 10⁵ host cells can be incubated in each well with a desired quantity of a test compound; in a 384-well plate, fewer than 10⁵ host cells and typically between 1×10⁴ and 4×10⁴ host cells are generally incubated in each well with the test compound.

The step of "measuring" can be achieved by different methods, the nature of which depends on the type of the reporter gene used. For example, the measurement can correspond to an optical density or a fluorescence emission in the case where the β-galactosidase, luciferase or green fluorescence protein gene is used as reporter gene.

The expression of the reporter gene can also be measured in terms of the hydrolysis of a substrate by the reporter gene expression product, such as for example a substrate of β-lactamase. One can mention in particular any product containing a β-lactam nucleus and the hydrolysis of which can be measured. Preferred substrates are those specific of β-lactamase (i.e., they are generally not hydrolyzed in mammalian cells in the absence of β-lactamase), those which are nontoxic to mammalian cells and/or whose hydrolysis product can be easily measured, for example by methods based on fluorescence, radioactivity, an enzymatic activity or any other method of detection.

In a general manner, the presence of the reporter gene product (or the hydrolysis product of a substrate from said reporter gene product) can be determined by conventional methods known to those skilled in the art [fluorescence, radioactivity, OD, luminescence, FRET (See. WO 00/37077), biochips, immunological methods, etc.]

The invention is particularly adapted to the selection of a large number of compounds which permit to enhance the level of expression of a gene implicated in antimicrobial defence, which compounds can be useful for treating and/or preventing microbial infection (e.g., *Shigella* infections).

Thus, in another preferred embodiment, the method according to the invention permits to select compounds which can be useful for treating and/or preventing a microbial infection, preferably for treating and/or preventing a *Shigella* infection, and more preferably for treating and/or preventing a *Shigella flexneri* infection.

The invention is also adapted for the selection of compounds which permit to enhance the level of expression of a gene implicated in antimicrobial defence, which genes are also tumour repressors such as DEFB103 as an example.

Thus, in another preferred embodiment, the method according to the invention permits to select compounds which can be useful for treating and/or preventing cancers.

The method according to the invention can further comprises a step e) of selecting the test compound which induces an enhanced level of expression of the reporter gene.

Said simple and efficient screening can be accomplished in a very short time. In particular, the described methods can be partially automated, thereby enabling the efficient and simultaneous screening of many different compounds, either in the form of a mixture or separately.

A secondary assay can be carried out whereby the selected compounds can be further validated, for example by determining their toxicity on cells culture or on animals, or by determining their efficiency, by example, on infected cells or animals.

Moreover, a second aspect of the invention consists in a compound identified by the method according to the present invention.

A third aspect of the present invention consists in a pharmaceutical composition comprising at least one compound identified by the method according to the present invention and, optionally, a pharmaceutically acceptable carrier.

As example of pharmaceutically acceptable carrier, the composition can comprise emulsions, microemulsions, oil-in water emulsions, anhydric lipids and water in oil emulsions, or other types of emulsions.

The composition of the invention may further comprises one or more additives such as diluents, excipients, stabilizers and preservatives. Such additives are well known to one skilled in the art and are for example, described in *"*Ullmann' Encyclopedia of Industrial Chemistry, 6th Ed." (various editors, 1989-1998, Marcel Dekker); and Pharmaceutical Dosage Forms and Drug Delivery Systems (ANSEL et al., 1994, WILLIAMS & WILKINS).

A fourth aspect of the invention consists in the use of at least one compound identified by the method according to the present invention for preventing and/or treating a microbial infection or for preventing and/or treating cancer in a subject.

As used herein, the term "subject" refers to a mammal, preferably to a human being.

One of skill in the art can simply determined the effective amount of said compound in order to obtain an antimicrobial activity.

In a preferred embodiment, the use of the invention is directed to preventing and/or treating a *Shigella* infection in a subject, and more preferably of a *Shigella flexneri* infection.

In the following, the invention is described in more detail with reference to nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### I. Preparation of bacterial strains

*Shigella flexneri* serotype 5a strains were isolated on Congo red agar plates. The invasive M90T wild-type strain (SANSONETTI et al., Infection and immunity, vol.35, p:852-860, 1982) and its isogenic derivatives, the invasive mxiE mutant (MAVRIS et al., Molecular microbiology, vol.43, p:1543-1553, 2002) (impaired for the MxiE transcriptional activator regulating expression of several virulence plasmid-encoded effectors), the non-invasive mxiD mutant (ALLAOUI et al., Molecular microbiology, vol.7, p:59-68, 1993) (impaired for the MxiD protein, a component of the TTSS required for its functionality) and the non-invasive plasmid-cured BS176 strain (SANSONETTI et al., abovementioned, 1982), were used. For infection experiments, strains were cultured overnight in BTCS medium (DIFCO) at 37°C with shacking. For human intestinal xenograft infections, overnight cultures were directly used. For polarized and differentiated monolayer cell infections, after fresh dilutions, subcultures were performed for 2 hours to reach the exponential phase, spun gently and used after resuspension of bacteria in tissue culture DMEM medium (INVITROGEN).

### II. Virulent Shigella modulates expression of specific innate immunity genes in vitro

### II.1. The wild-type Shigella impairs expression of specific innate immunity genes by injecting virulence factors into epithelial cells through its TTSS

To investigate whether *Shigella* modulates expression of genes involved in the intestinal innate immune response, such as the genes encoding antimicrobial factors, an *in vitro* model of infection based on polarized and differentiated TC7 or HT29 colonic epithelial cells was established.

This model mimics the epithelial lining and allows detection of transcripts for antimicrobial factors whose expression are correlated with the degree of cellular differentiation (HASE et al., Infection and immunity, vol.70, p:953-963, 2002).

TC7 (CHANTRET et al., Journal of cell science, vol.107 ( Pt 1), p:213-225, 1994) and HT29 epithelial cells derived from a colonic carcinoma were cultured in 6-well plates (1.5×10⁶ cells/well) with DMEM (INVITROGEN) supplemented with 10% FCS (INVITROGEN), 1% non-essential amino acids (INVITROGEN), 100 U/ml penicillin and 100 µg/ml streptomycin (INVITROGEN), in 10% CO2. To obtain polarized and differentiated cell monolayers, cells were grown for 3 weeks to reach full differentiation and polarization. Culture medium was changed three times per week.

Infections were performed on cell monolayers for 1 h 30 with the invasive wild-type *Shigella flexneri* strain M90T and its isogenic derivatives: the invasive mxiE mutant, the non-invasive mxiD mutant, and the non-invasive plasmid-cured BS176 strain. The mxiE mutant is impaired for the MxiE transcriptional activator regulating expression of several virulence factors, such as the Osp and IpaH proteins, encoded by the virulence plasmid and injected into cells upon infection, whereas the mxiD mutant is impaired for the assembly of a functional TTSS.

To facilitate *Shigella* infection, the cell culture plates were spun gently for 10 min at 1000 rpm at room temperature and then placed at 37°C.

After 1 h 30 of infection, 50 µg/ml gentamicin antibiotic (INVITROGEN) was added in order to stop the infection. As a control, lactate dehydrogenase was used (CYTOTOX, PROMEGA).

Host cell RNAs were harvested at 1 h 30, 3 h and 4 h 30 post-infection, washed with phosphate buffered saline (PBS) and then lysed for RNAs isolations. qRT-PCR analysis were performed for the expression of a selected set of innate immunity genes.

RNAs were isolated using the RNEASY MINI kit (QIAGEN). Quality and quantity were determined using the AGILENT nanochip technology (AGILENT). Reverse transcription-PCR reactions were performed on 4 µg RNA samples using the SUPERSCRIPT II REVERSE TRANSCRIPTASE (INVITROGEN) and the oligo(dT)15 primers (PROMEGA) as recommended by suppliers. Gene specific primers were purchased from PROLIGO (SIGMA-GENOSYS): HBD1 5'-CAGGTGGTAACTTTCTCACAGG-3' (SEQ ID NO:5) / 5'-AATAGAGACATTGCCCTCCACT-3' (SEQ ID NO: 6), HBD2 5'-GCCATGAGGGTCTTGTATCTC-3' (SEQ ID NO: 7) / 5'-TTAAGGCAGGTAACAGGATCG-3' (SEQ ID NO: 8), HBD3 5'-GTGTGTTCTGCATGGTGAGAG-3' (SEQ ID NO: 9) / 5'-TATAAAGGTTCCAGCCACAGC-3 (SEQ ID NO: 10)', LL37 5'-CGGAAATCTA AAGAGAAGATTGG-3' (SEQ ID NO: 11) / 5'-TAGGGCACACACTAGGACTCTG-3' (SEQ ID NO: 12), CCL20 5'-GACATAGCCCAAGAACAGAAAG-3' (SEQ ID NO: 13) / 5'-TAATTGGACAAGTCCAGTGAGG-3' (SEQ ID NO: 14), CCR6 5'-CAGTCATCATCTCCAGCTCAAC -3' (SEQ ID NO:15) / 5'-CTCCGAGACAGTCTGGTACTTG -3' (SEQ ID NO:16),.

Quantitative RT-PCRs were carried-out in a 20 µl volume containing 8 µl of cDNA (diluted at 1/100), specific primers (0,2 µM each) and 10 µl of Power SYBR GREEN mix (APPLIED BIOSYSTEMS). Reactions were run on an ABI 7900HT instrument (APPLIED BIOSYSTEMS) with conditions of the recommended universal thermal cycling parameters. Each reaction was run in duplicate. Relative quantification of gene expression was performed using the comparative Ct method. Results were normalized using the human β-2-microglobulin (B2M) housekeeping gene.

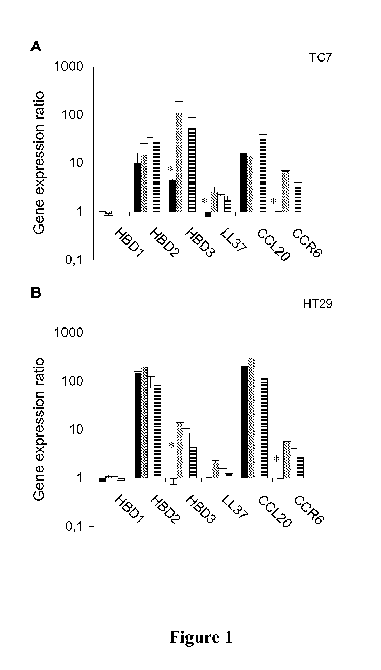

The figure 1 A and B show the expression of β-defensins HBD1, HBD2 and HBD3, cathelicidin LL37, chemokine CCL20, and chemokine receptor CCR6 encoding genes in TC7 (figure 1A) or HT29 (figure 1B) cell monolayers 4 h 30 post-infection (3 h after gentamicin addition). After RNAs extractions and reverse transcription reactions, qRT-PCR were performed on each samples with specific primers to determine the relative expression of genes in each conditions, using the comparative Ct method. The results are presented on a logarithmic scale as the ratio of gene expression in infected cells compared to non-infected cells. Black bars: wild-type strain M90T; gray bars: plasmid-cured BS176 strain; white bars: mxiD mutant strain; hashed bars: mxiE mutant strain. N represents 3 independent biological experiments. Error bars represent the SD. Stars: P < 0,01 for infections with the wild-type strain compared to the other strains.

The results show that in non-infected cell monolayers, a basal expression of β-defensins HBD1, HBD2 and HBD3, cathelicidin LL37, chemokine CCL20 and chemokine receptor CCR6 encoding-genes was observed. In contrast, no detection of transcription of the β-defensin HBD4 gene in all tested conditions, including infection experiments was observed.

The results further established that infection of TC7 or HT29 cells with the non-invasive mxiD or BS176 strains was followed by a strong transcriptional induction of HBD2, HBD3, LL37, CCL20 and CCR6 genes, over their basal level that could be observed as early as 1 h 30 following infection, whereas the HBD1 gene remained expressed at its basal level of expression throughout the time course experiments (Fig. 1A).

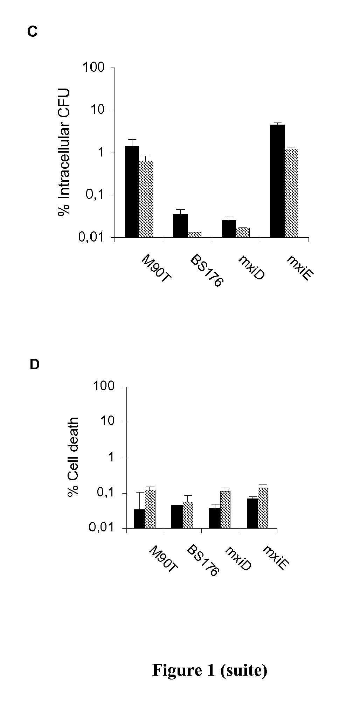

The figure 1C shows *Shigella* infection experiments on polarized and differentiated TC7 or HT29 epithelial cells. The gentamicin assays were performed in conditions similar to those used for infection experiments (1.5×10⁶ cells/well; MOI: 100; time course: 1 h 30). The values represent the percentage of intracellular CFU. Black bars represent TC7 cells and grey bars represents HT29 cells. N represents 3 independent biological experiments. Finally, the error bars represent the SD.

The figure 1D shows the cellular viability for TC7 or HT29 cell monolayers infected with the wild-type *Shigella* strain and its derivatives. The lactate dehydrogenase assays were performed in 6-well plates (1.5×10⁶ cells/well; MOI: 100; time course: 1 h 30). Black bars represent TC7 cells and grey bars represents HT29 cells. Values represent the percentage of cell death. N represents 3 independent biological experiments. Error bars represent the SD.

The results show that the invasive wild-type strain M90T was unable to significantly invade polarized and differentiated cell monolayers, as assessed by gentamicin assays (Fig. 1C), in agreement with data obtained in apically-infected polarized Caco-2 cells (MOUNIER et al., abovementioned, 1992).

However, the previous results have established that infection experiments with the strain M90T have shown differential transcriptional expression of the cellular genes HBD3 and CCR6 in both cell types, and for LL37 gene in TC7 cells only, compared to infections with the non-invasive mxiD and BS176 strains (Fig. 1A and B). At 4 h 30 post-infection, transcription of these genes in infected cells was induced to a lesser extent by the wild-type strain M90T compared to the mxiD and BS176 strains. In TC7 cells, the HBD3 gene expression was induced only 4 fold by M90T compared to more than 110 fold by BS176 (Fig. 1 A). Conversely, expression of HBD1, HBD2 and CCL20 genes was found similar for all infections with the two cell lines (Fig. 1 A and B). As a control, it has been established that the cellular viability was the same for monolayers infected with the wild-type *Shigella* strain and its derivatives, as assessed by lactate dehydrogenase assays (Fig. 1D).

Finally, these results taken together indicate that wild-type *Shigella* impairs expression of specific innate immunity genes by injecting virulence factors into epithelial cells through its TTSS, thereby affecting the host immune response.

### II. 2. The pool of MxiE-dependent effectors is involved in the host innate immunity genes downregulation

The *Shigella* MxiE regulon includes more than ten virulence proteins delivered through the TTSS upon contact of the bacteria with cells. Among them, the OspF and OspG effectors have been shown to target the host NF-κB and mitogen-activated protein kinases (MAPKs) signaling pathways (ARBIBE et al., Nature immunology, vol.8, p:47-56, 2007; KIM et al., Proceedings of the National Academy of Sciences of the United States of America, vol.102, p:14046-14051, 2005; KRAMER et al., PLoS pathogens, vol.3, p:e21, 2007; LI et al., Science (New York, N.Y), vol.315, p:1000-1003, 2007). Expression of the genes encoding these proteins is dramatically affected in a mxiE mutant, therefore preventing their synthesis and injection into cells upon infection ( LE GALL et al., Microbiology (Reading, England), vol.151, p:951-962, 2005). However, the mxiE mutant still has a functional TTSS for injection of the MxiE-independent effectors, allowing an efficient invasion of non-polarized epithelial cells, comparable to the M90T wild-type strain (LE GALL et al., abovementioned, 2005; MAVRIS et al., abovementioned, 2002).To determine whether the pool of MxiE-dependent effectors was involved in the host innate immunity genes downregulation, the same infection experiments as described above were conducted with the invasive mxiE mutant.

As observed previously for the wild-type strain M90T, the mxiE mutant was unable to significantly invade polarized and differentiated TC7 or HT29 cells s (Fig. 1C). However, in contrast to M90T, the mxiE strain was unable to modulate transcription of HBD3, LL37 and CCR6 genes in infected cell mono layers (Fig. 1A and B). Transcription of these genes, as well as the other genes studied, was similar in cells upon mxiE-, mxiD- or BS176-infection (Fig. 1A and B).

In addition, infection experiments performed with mutant strains defective for the *ospF* or *ospG* genes revealed that the encoded effectors repressed antimicrobial factor genes transcription. However, the extent of repression was inferior to the one observed with the whole set of effector proteins delivered by the M90T strain, suggesting additive synergistic action of these effectors to get full repression of the antimicrobial peptide genes expression.

Thus, the pool of MxiE-dependent effectors injected in cells upon infection is responsible for the observed manipulation of the host immune response through transcriptional dampening.

### II.3. Shigella is able to modulate transcription of innate immunity genes in cells induced to undergo an inflammatory response pattern

Expression of several genes from the innate immune response is known to be upregulated, in intestinal epithelial cells, by treatment with pro-inflammatory molecules such as IL-1β, TNF-α or IFN-γ (SELSTED & OUELLETTE, abovementioned , 2005).

In order to determine whether *Shigella* was still able to modulate transcription of innate immunity genes in cells induced to undergo an inflammatory response pattern, TC7 cells monolayers were stimulated by 20 ng/ml of IL-1β (R&D Systems) for 2 hours, the IL-1β-containing medium was then removed and *Shigella* infections were performed as described above.

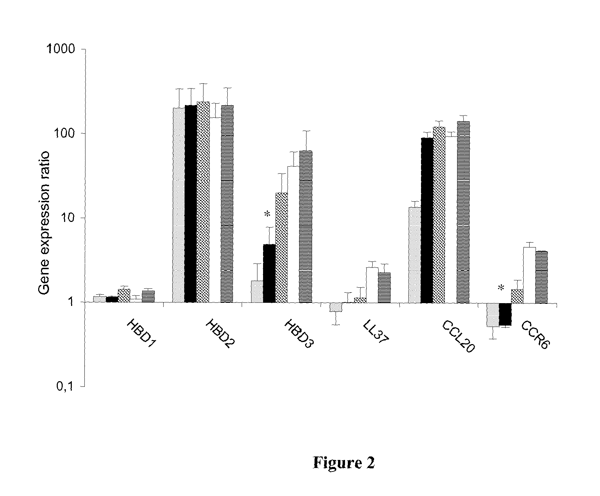

The figure 2 shows the expression of β-defensins HBD1, HBD2 and HBD3, cathelicidin LL37, chemokine CCL20, and chemokine receptor CCR6 encoding genes at 3 h after gentamicin addition in TC7 cells pre-stimulated by IL-1β. After RNAs extractions and reverse transcription reactions, qRT-PCR were performed on each samples with specific primers to determine the relative expression of genes in each conditions, using the comparative Ct method. The results are presented on a logarithmic scale as the ratio of gene expression in stimulated and infected cells compared to non-stimulated and non-infected cells. Dotted bars: non-infected; black bars: wild-type strain M90T; gray bars: plasmid-cured BS176 strain; white bars: mxiD mutant strain; hashed bars: mxiE mutant strain. N represents 3 independent biological experiments. Error bars represent the SD. Stars: P < 0,01 for infections with the wild-type strain compared to the other strains..

The results show that IL-1β treatment was followed by an induction of the HBD2, HBD3 and CCL20 genes transcription in non-infected cells (Fig. 2). In the same conditions, expression of the HBD1 gene remained unchanged, whereas a weak downregulation was observed for CCR6 gene expression (Fig. 2).

Interestingly, the wild-type *Shigella* strain M90T conserved its capacity to actively repress HBD3 gene expression in IL-1β stimulated cells (Fig. 2).

As expected, the mxiE mutant strains as well as the mxiD and BS176 strains were still unable to modulate HBD3 gene induction in IL-1β treated cells (Fig. 2).

Similarly, wild-type bacteria repressed CCR6 gene transcription in stimulated cells, whereas mxiD and mxiE mutants failed to dampen its expression in similar conditions (Fig. 2).

Taken together, these *in vitro* results indicate that virulent *Shigella* manipulates the host innate immune response, through injection of the MxiE-dependent effectors, even in cells expressing an inflammatory response pattern, a situation that is likely to prevail regarding the dynamics of interactions between luminal bacteria and the epithelial lining in the course of an infection.

### III. Antimicrobial factors whose transcription is repressed by Shigella are those affecting highest bactericidal activity against the pathogen

### III. 1. β-defensin hBD-3 exhibited a strong bactericidal activity on Shigella

In order to evaluate the antimicrobial activity of innate immune factors such as β-defensins hBD-1, hBD-2 and hBD-3, cathelicidin LL37 and chemokine CCL20 on *Shigella* viability, the *in vitro* antimicrobial activity of each of these peptides against the pathogen, using synthetic or recombinant molecules was determined.

Antimicrobial activities of the synthetic human β-defensins hBD-1, hBD-2, hBD-3 (PEPTANOVA), cathelicidin LL37 (PHOENIX PHARMACEUTICALS), and the recombinant chemokine CCL20 (PEPROTECH) were tested on the different *Shigella flexneri* strains, as described before (JEPRAS et al., Antimicrobial agents and chemotherapy, vol.41, p:2001-2005, 1997).

Briefly, fresh dilutions of over-night *Shigella* cultures were subcultured for 2 hours, bacteria were spun gently, resuspended in DMEM medium (INVITROGEN) containing 5 µg/ml (CCL20) or 50 µg/ml (hBD1-3 and LL37) of the relevant peptide, and incubated for 2 hours at 37°C with intermittent shaking, in a 100 µl final volume. Because the activity of most antimicrobial factors is antagonized by high salt concentrations, DMEM medium was diluted to 1:2 for these experiments (salts 0,08 M; pH 7,2; no serum) (SELSTED & OUELLETTE, abovementioned, 2005). Bacteria were then incubated for 10 min at room temperature with 10 µg/ml bis-(1,3-dibutylbarbituric acid)-trimethine oxonol fluorescent molecule (DiBAC4, MOLECULAR PROBES), which crosses depolarized bacterial membrane only, before acquisition. Analysis of DiBAC4-fluorescent bacteria was performed with a FACsCalibur apparatus (BD BIOSCIENCES) using the CELLQUESTPRO software. The mean fluorescence is presented in arbitrary fluorescent units (a.f.u.).

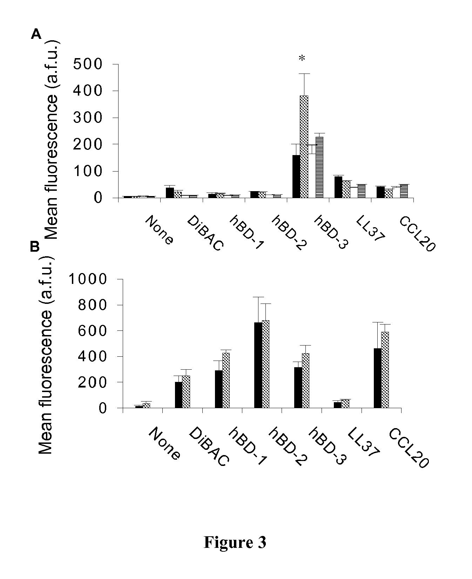

The figure 3A shows the bactericidal activities of synthetic β-defensins hBD-1, hBD-2 and hBD-3, cathelicidin LL37, or recombinant chemokine CCL20 on *Shigella* exponential cultures growing in DMEM medium for 2 hours at 37°C. The values expressed in arbitrary fluorescence units (a.f.u.) represent the mean fluorescence of the bacterial population analyzed. Black bars: wild-type strain M90T; gray bars: plasmid-cured BS176 strain; white bars: mxiD mutant strain; hashed bars: mxiE mutant strain. N represents 3 independent experiments. Error bars represent the SD. Stars: P < 0,01 for the fluorescence obtained with the BS 176 strain compared to the other strains.

The figure 3B shows the antimicrobial activity of synthetic β-defensins hBD-1, hBD-2 and hBD-3, cathelicidin LL37, or recombinant chemokine CCL20 on *Wisteria monocytogenes* and *Listeria innocua* exponential cultures. Black bars: *L. monocytogenes;* grey bars: *L. innocua.* N represents 3 independent experiments. Error bars represent the SD.

As assessed by the weak fluorescence values obtained (Fig. 3A), the results established that β-defensins hBD-1 and hBD-2 were inactive on the *Shigella* wild-type strains and its derivatives. Conversely, the β-defensin hBD-3 exhibited a strong bactericidal activity on the pathogen at a similar concentration (Fig. 3A).

Interestingly, the plasmid-cured BS176 strain appeared 2-fold more sensitive to hBD-3 than the other strains, as confirmed by CFU assays , suggesting that protein(s) encoded by the virulence plasmid achieve higher bacterial resistance to this antimicrobial factor.

Finally, among the antimicrobial factors tested, cathelicidin LL37 and chemokine CCL20 exhibited an intermediary antibacterial action on *Shigella* compared to hBD-1/hBD-2 and hBD-3 (Fig. 3A). As a control of our experimental conditions, *Listeria monocytogenes* and *Listeria innocua* were tested and found sensitive to all the antimicrobial peptides tested excepted cathelicidin LL37, at equivalent concentrations (Fig. 3B).

### III. 2. Shigella is unlikely to proteolytically degrade the antimicrobial factors.

In order to explore the possibility of plasmid-encoded proteolytic degradation of hBD-3, the activity of the defensin in the presence of *Shigella* mutants defective for the expression of the surface/secreted proteases SepA or SopA that are encoded by the virulence plasmid, and the sepA-BS176 complemented strain, was tested (SANSONETTI & EGILE, vol.74, p:191-197, 1998; BENJELLOUN-TOUIMI et al., Microbiology (Reading, England), vol.144 ( Pt 7), p:1815-1822, 1998). The results have established that no change in bacterial sensitivity was observed with any of the mutant strains, suggesting that *Shigella* is unlikely to proteolytically degrade the antimicrobial factors.

Collectively, these results show that among the molecules tested *in vitro,* the most active antimicrobial factors for *Shigella* are those whose expression is suppressed by the virulent bacterium at the transcriptional level.

### IV. Shigella regulates expression of innate immunity genes in vivo

In order to confirm *in vivo* that virulent *Shigella* downregulates expression of antimicrobial factors, a transcriptomic analysis of xenotransplanted human intestinal segments following infection for 2 hours by the wild-type strain M90T, the mxiE mutant and the avirulent strain BS176 was performed.

Human intestinal xenografts were placed into the subscapular region of 6-8 week old SCID mice as previously described (SEYDEL et al., Infection and immunity, vol.65, p:1631-1639, 1997). Grafts were allowed to develop for 10 weeks before use, then human tissues on mice were infected by direct intraluminal inoculation with 5×107 *S*. *flexneri* bacteria.

A group was mock infected by inoculation with media alone as a control.

At 2 hours post-infection, animals were sacrificed, and grafts were removed and frozen for Trizol RNA isolation (Invitrogen), or for immuno-histological analysis. In the later case, they were embedded on Optimal Cutting Temperature (OCT) compound (Sakura) and frozen in isopentane-dry ice.

Animal experiments were done according to the guidelines of the Institut Pasteur's ethical committee for animal use in research.

Infection of these tissues for short time periods allowed for study of gene transcription in human epithelial cell, thanks to the use of human-specific arrays.

After infections, a section of each graft was removed and homogenized with an Ultra-Turrax apparatus (JANKE & KUNKEL) in 2 ml of Trizol (INVITROGEN). Total RNA was extracted according to the manufacturer's instructions, and cleaned by a subsequent purification using the RNEASY MINI kit (QIAGEN). RNAs quantity and quality were analyzed by the Agilent Bioanalyzer (AGILENT). The cRNA synthesis, hybridization and labeling were done as described before (PEDRON et al., The Journal of biological chemistry, vol.278, p:33878-33886, 2003). 10 µg of cRNA were fragmented and hybridized to the Affymetrix U133A GeneChip Human Array (AFFYMETRIX). After PE-Streptavidin staining, arrays were scanned at 488 nm using an argon-iron laser. Data were analyzed with the SPLUS ARRAYANALYSER software (INSIGHTFUL1). Preprocessing by Robust Multi-chip Average (RMA) was applied to process individual probe values (perfect match) and to generate summary values for each probe set (transcript) (IRIZARRY et al., Biostatistics (Oxford, England), vol.4, p:249-264, 2003). As two replicates were done, statistical analyses were performed using the Local Pool Error test, an algorithm dedicated to small number of replicate arrays (JAIN et al., Bioinformatics (Oxford, England), vol.19, p:1945-1951, 2003). The p-values were adjusted using the BONFERRONI algorithm. The dChip software was used for hierarchical clustering with Euclidean distance and average as a linkage method (LI &. WONG, Genome informatics, vol.12, p:3-13, 2001). Before clustering, expression values for one gene across all samples were standardized to produce a mean of zero. Increased or decreased values were then ranged compared to that mean. GEO database accession code: microarray data, GSE8636.

Two independent array hybridization experiments were performed for each condition and the hierarchical clustering of modulated genes expression was determined. Validation of data was performed on a set of genes, including HBD2 and HBD3 whose probe-sets were absent from the used arrays, using qRT-PCR.

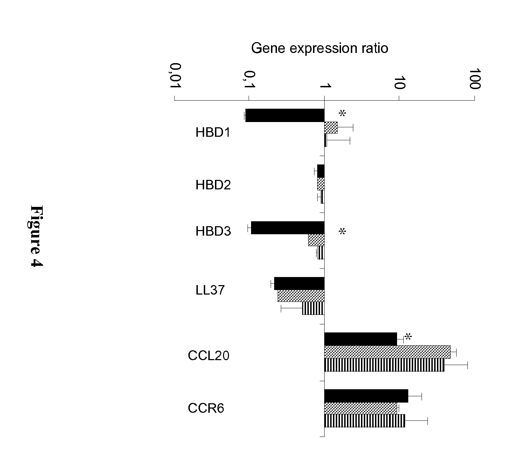

The figure 4 illustrates validation/determination by qRT-PCR of β-defensins HBD1, HBD2 and HBD3, cathelicidin LL37, chemokine CCL20, and chemokine receptor CCR6 genes expression in infected tissues used for GENECHIP experiments. After reverse transcription reactions, qRT-PCR were performed on each samples with specific primers to determine the relative expression of genes in each conditions using the comparative Ct method. The results are presented on a logarithmic scale as the ratio of gene expression in infected tissues compared to non-infected tissues. Black bars: wild-type strain M90T; gray bars: plasmid-cured BS176 strain; hashed bars: mxiE mutant strain. N represents 2 independent biological replicates per condition. Error bars represent the SD. Stars: P < 0,01.

From this genes cluster, a total of 46 genes whose expression was significantly modulated by *Shigella* in infected intestinal xenotransplants was identified.

Interestingly, 11 genes were less transcribed upon infection with the wild-type strain M90T, compared to the invasive mxiE mutant, or the non-invasive BS176 strain (Fig. 4). These targets of the MxiE-dependent effectors included genes encoding chemokines and chemokine receptors such as CCL3, CCL4, CCL20, CCL25 and CCRL2. Mainly produced by epithelial cells, these proteins are involved in the recruitment and activation of inflammatory and immune effectors cells such as macrophages, dendritic cells and T cells (DIEU-NOSJEAN et al., Journal of leukocyte biology, vol.66, p:252-262, 1999). Expression of genes encoding cytokines IL7 and IL18, and cytokine receptors IL13RA1 and IL20RA was also less expressed in human tissues infected by the M90T strain, compared to the mxiE mutant. IL7 is produced locally by intestinal epithelial and epithelial goblet cells, and may serve as a regulatory factor for intestinal mucosal lymphocytes, whereas IL18 induces IFN-γ production by T cells.

Finally, among downregulated genes, as measured by qRT-PCR, expression of β-defensins HBD1 and HBD3 was strongly reduced in xenotransplants infected by wild-type bacteria compared to mxiE mutants: ratios 0.09 vs 1.09 for HBD1, and 0.1 vs 0.82 for HBD3, respectively (Fig. 4).

In addition to downregulating the expression of several genes encoding chemokines, cytokines and antimicrobial peptides, transcriptional analysis indicated that MxiE-dependent bacterial effectors upregulate the expression of the SOCS2 gene (Fig. 4A). Interestingly, members of this family are cytokine-inducible negative regulators of cytokine signaling pathways whose primary function is to attenuate the JAK/STAT signaling pathway. In turn, a potential role of the JAK/STAT signaling pathway in downregulating expression of HBD1 and HBD3 has been suggested (JOLY et al., Molecular immunology, vol.42, p:1073-1084, 2005).

Taken together, these *in vivo* results showed that *Shigella* is able to manipulate the host innate immune response, through its MxiE-dependent effectors, by up- or downregulating expression of crucial innate immunity genes.

### V. Shigella blocks antimicrobial factors expression in vivo

The *in vivo* consequences of this strategy in intestinal tissues infected by the pathogen were investigated.

### V.1. Shigella causes mucosal lesions

Xenotransplants were infected for 2 hours with the wild-type strain M90T, the mxiE mutant, the non-invasive mxiD mutant harboring an inactive TTSS, or injected with bacterial culture medium alone as control.

Hematoxyline staining revealed that mxiE mutant caused less mucosal lesions than wild-type bacteria, showing only some areas of epithelial destruction and abscesses

Conversely, no significant alteration of tissue structures was observed in xenotransplants infected with non-invasive mxiD mutants, as compared to non-infected tissue.

*S. flexneri* 5a LPS immunostaining revealed that M90T wild-type bacteria were distributed more diffusely in the mucus layer than mxiE mutants, whose localization remained close to the top of the villi.

In addition, mxiE mutants were rarely seen localized in lumenal intervilli spaces near the cryps, compared to wild-type bacteria that affected a deeper diffusion pattern. Finally, the presence of M90T and mxiE strains was observed in the lamina propria, in a few occasions at this early time point of infection.

### V.2. Shigella causes poor hBD-1, hBD-3 and CCL20 synthesis

Immunolabelings were performed on infected tissues with antisera to human β-defensins hBD-1, hBD-3 and chemokine CCL20.

Cryostat sections (7 µm) were prepared from human intestinal xenografts conserved in OCT. Sections were rehydrated, blocked for 30 min in PBS with 1% BSA, and immunostained with the following antibodies: polyclonal antibody to *Shigella flexneri* 5a LPS, polyclonal antibody to human hBD-1 (FL-68; SANTA CRUZ BIOTECHNOLOGY), polyclonal antibody to human hBD-3 (FL-67; SANTA CRUZ BIOTECHNOLOGY), polyclonal antibody to human CCL20 (AF360; R&D Systems). Slides were washed twice in PBS before addition of the appropriate peroxidase-conjugated secondary antibody. Detection was accomplished by addition of the Dako liquid DAB Substrate-Chromogen System (DAKO).

Non-infected tissues revealed weak staining for hBD-1 and no labeling for hBD-3 and CCL20, in agreement with the respective constitutive and inducible production of these molecules by epithelial cells *in vivo*.

Interestingly, in tissues infected by the wild-type strain M90T, hBD-1 and hBD-3 staining exhibited a weak labeling following the villi contours, suggesting poor synthesis and release of these molecules in the lumen.

Conversely, in mxiE mutant-infected tissues, massive luminal release of these antimicrobial peptides was observed from epithelial cells exposed to the colonizing bacteria, imprinting the portion of the mucus layer proximal to the epithelial surface.

As expected for their profile, β-defensin hBD-2 immunostaining was similar in human xenografts infected by wild-type and mxiE mutant bacteria.

In agreement with the transcriptional data from xenografts, CCL20 labeling showed strong production of this chemokine by enterocytes along villi with luminal efflux in the transplants infected by the mxiE strain, whereas only a weak signal was detected in tissues infected by the M90T strain.

Other defensins- and chemokine-staining experiments indicated a similar staining pattern for mxiD- compared to mxiE-infected xenografts . Taken together, these experiments indicate that MxiE-dependent *Shigella* effectors affect the production of hBD-1, hBD-3 and CCL20, molecules exhibiting antimicrobial activities, thereby weakening the antimicrobial defence barrier at infected mucosal surfaces.

### V.3. Blocking of antimicrobial factors expression correlates with deeper progression of Shigella towards intestinal crypts

The progression of *Shigella* towards intestinal crypts of infected xenograft tissues was evaluated by determining and comparing the amount of bacteria from the top of the villi to the crypts of the mucosa.

Briefly, sections from xenograft tissues infected by the wild-type *Shigella* strain M90T, and the mxiD and mxiE mutants, were immunostained with a polyclonal antibody to *Shigella* 5a LPS, and used to count the bacteria localized at the top of the villi (upper third), in the luminal intervilli spaces (intermediate third), and in the crypts (lower third) of the mucosal tissues. A representative set of 10 villi per section was systematically analysed and 3 sections were read for 4 independent infected xenograft tissues. Data are presented as a percentage of the amount of bacteria. The 100% is defined by the amount of M90T bacteria determined at the upper third of the villi.

The results show that at 2 hours post-infection, the presence of the M90T wild-type strain and the mxiE mutant in the *lamina propria* of infected xenotransplants was observed only in few occasions. However, the strong epithelial expression of antimicrobial factors observed at the same time indicates the establishment of a mucosal inflammatory response.

Thus, it was studied how the epithelial inflammatory response affected the behaviour of bacteria at the mucosal surface, especially their ability to progress through intervilli spaces, towards crypts. For this purpose, a qualitative and quantitative analysis of the bacterial distribution from the top of the villi to the crypts of xenotransplants infected for 2 hours with the wild-type strain M90T, or the mxiE and mxiD mutants was performed.

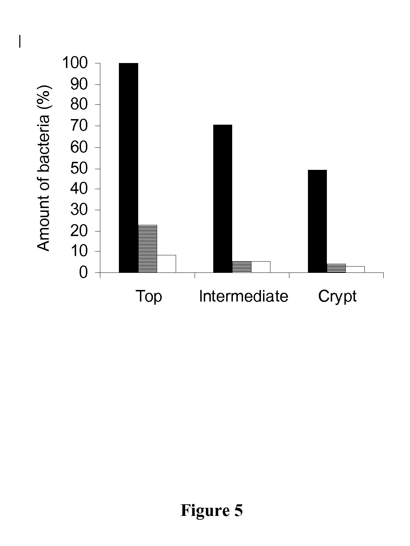

Figure 5 illustrates bacterial counts in the mucosal tissue. The anti-*Shigella* 5a LPS immunostaining sections were used to count the bacteria localized at the top of the villi (upper third), in the luminal intervilli spaces (intermediate third), and in the crypts (lower third). A representative set of 10 villi per section was analyzed and 3 sections were read for 4 independent biological replicates per condition. The results are presented as a percentage of the amount of bacteria found at each location. The 100% is defined by the amount of M90T bacteria determined at the upper third of the villi. Black bars represent wild-type strain M90T, hashed bars represent mxiE mutant strain and white bars represent mxiD mutant strain. N represents 4 independent biological replicates per condition.

Qualitatively, *S. flexneri* 5a LPS immunostainings revealed that wild-type bacteria were distributed more diffusely in the mucus layer than mxiE and mxiD mutants, whose localization remained close to the top of the villi, essentially trapped in the luminal mucus. In addition, mxiE and mxiD mutants were barely seen localized in luminal intervilli spaces or deep near the crypts, compared to wild-type bacteria that affected a much deeper diffusion pattern. Quantitatively, the amount of M90T bacteria observed in the lower third of the villi, including the crypt areas, was close to the half of the one determined at the upper third (Fig. 5).

Collectively, these results highlight the correlation existing between the ability of virulent *Shigella* to block antimicrobial factors expression, and their capacity to progress deeply and massively towards intestinal crypts, at the early time point of infection.

### V. 4. Shigella compromises recruitment of DCs to the lamina propria of infected tissues

To correlate these findings to mouse immune cell trafficking, additional immunostaining experiments were performed using a monoclonal antibody to mouse CD11c (HL3; BDBiosciences), one of the main antigens present on the surface of dendritic cells.

CD11c staining of mxiE-infected tissues revealed massive recruitment of DCs into the *lamina propria* and submucosal regions, probably attracted by the gradient of chemotactic molecules, such as CCL20, emanating from epithelial cells. Similar observations were obtained in mxiD-infected tissues (data not shown ).

In contrast, M90T-infected xenotransplants showed a very different pattern characterized by a restricted presence of DCs in submucosal areas as well as a poor migration into the lamina propria with much less staining than observed in non-infected tissues for resident DCs.

Taken together, these experiments highlight the existence of a dedicated MxiE-dependent system allowing *Shigella* to suppress expression of immune effectors, leading to compromised recruitment of DCs to the *lamina propria* of infected tissues.

### VI. Screening method to identify compounds enhancing the expression of the previously identified genes related to Shigella immune escape strategy

### VI.1. Vectors construction

The construction of recombinant lentivirus comprising each of the promoters of interest was based on VIRA POWER™ Promoterless Lentiviral GATEWAY® KIT (INVITROGEN).

PCR reactions were performed on TC7 genomic DNA using primers specific for the DEFB4 (hBD-2), DEFB103 (hBD-3), CAMP (LL37) and CCL20 promoter regions.

The primers containing engineered restriction sites in their sequences were as follow:
*DEFB4: 5'CACTGTACCACCAGTAGCAATAACCG 3' (SEQ ID NO: 17)
   5'CTGATGGCTGGGAGCTTCACCAGGAG 3' (SEQ ID NO: 18)
*DEFB103: 5' GAGGCACATGTTGCAAAACGGTCAGG 3' (SEQ ID NO: 19)
   5' CTGCTAGGCTTCACCCCACGCTGAGA 3' (SEQ ID NO: 20)
*CAMP: 5'GAGTGGGAGAGGGTAGCTCCAGCATC 3'(SEQ ID NO: 21)
   5' CATGTCTGCCTCCCTCTAGCCCACAG 3'(SEQ ID NO: 22)
*CCL20: 5' TCTACAAAGAAGCCAGCCAGGATTTT 3'(SEQ ID NO: 23)
   5' GACAGCAGCCTGGGATGGCCCTATTT 3'(SEQ ID NO: 24)

The DNA products corresponding to the previously disclosed promoters were then cloned in pENTR/5'-TOPO vector using pENTR™5'-TOPO®TA Cloning ® Kit (INVITROGEN) by a 5-minute bench-top ligation according to the manufacturer's instruction.

Chemically competent *E. coli* were transformed with the TOPO® Cloning reactions. The transformants were then selected on kanamycine. The plasmids of interest were then purified using PURELINK™HiPure Plasmid Midiprep Kit (INVITROGEN) according to manufacturer's instruction.

Simultaneously, the eGFP reporter gene was cloned in pENTR^{™}/D-TOPO ® vector using pENTR^{™}/D-TOPO ® Cloning ® Kit (INVITOGEN) according to the manufacturer's instruction. The plasmid of interest was then purified using PURELINK^{™}HiPure Plasmid Midiprep Kit (INVITROGEN) as previously.

A recombination reaction was then performed for obtaining each recombinant lentivirus of interest comprising eGFP under the control of each promoter of interest - i.e., DEFB4, DEF, CAMP, and CCL20-.

For each promoter, 10 fmoles of pENTR/5'-TOPO promoter vector, 10 fmoles of pENTR^{™}/D-TOPO eGFP vector and 20 fmoles of pLenti6/R4R2/V5-DEST in 8 µl final volume were mixed with 2 µl of LR Clonase ^{™} II Plus enzyme mix (INVITROGEN). The reactions were then incubated overnight at room temperature.

The reactions were stopped by addition of Proteinase K and were used to transformed One Shot® Stb13^{™} chemically competent *E. coli* (INVITROGEN).

The transformants were then selected using 100 µg/ml ampicillin (SIGMA) and 50 µg/ml Blastidicin (SIGMA).

Each lentiviral DNA was then purified and sequenced.

Each of the purified pLenti6/R2R4/V5-DEST expression plasmid comprising the promoter DEFB4, DEFB103, CAMP or CCL20 and the eGFP gene was resuspended in TE Buffer (pH 8.0) to a final concentration of 1 µg/µl.

In a sterile 5ml tube, 9 µg of the ViraPower^{™} Mix and 3 µg of pLenti-based plasmid DNA were diluted in 1.5 ml of Opti-MEME® I Medium without serum and mixed gently. This operation was done for each pLenti plasmid.

In another sterile 5 ml tube, 36 µl of LIPOFECTAMINE^{™} 2000 were diluted in 1.5 ml of Opti-MEME® I Medium without serum and mixed gently.

After 5 min incubation, the diluted DNA and diluted LIPOFECTAMINE^{™} 2000 were combined and incubated 20 minutes at room temperature.

The DNA-lipid complexes were then used to transformed 6 x 10⁶ 293FT cells, which were incubated overnight at 37°C in a CO₂ incubator. The next day, the medium was replaced with complete culture medium containing sodium pyruvate.

Finally, the virus-containing supernatants were harvested 48 hours posttransfection, centrifuged at 3,000 rpm for 15 minutes at 4°C to pellet cell debris and stored at -80°C.

### VI.2. Cells infection

TC7 and U937 cells were transduced with each lentiviral construct and the transformants were selected using Blasticidin.

### VI.3. Screening of compounds with potential antimicrobial activity

TC7 and U937 cells expressing eGFP under the control of DEFB4, DEFB103, CAMP or CCL20 promoter were contacted with different compounds. The inventors select the compounds inducing the expression of eGFP as potential antimicrobial and/or anti-tumoral agents.

As a positive control of DEFB4 promoter inducer, IL-1β is used (SINGH et al., Proc. Natl. Acad. Sci. U.S.A., vol.95(25), p:14961-6, 1998).

As a positive control of DEFB103 promoter inducer, IFNγ is used (HARDER et al., J. Invest. Dermatol., vol.123(3), p:522-9, 2004)

Finally, and as a positive control of CAMP promoter inducer, butyrate is used (SCHAUBER et al., Gut, vol.52(5), p:735-41, 2003).

## Claims

1. Method for the selection of compounds which permit to enhance the level of expression of a gene implicated in antimicrobial defence comprising the step of:
a) contacting a test compound with an host cell expressing a reporter nucleic acid comprising a nucleic acid sequence coding for a reporter placed under the control of a promoter, which promoter comprises all or part of the promoter sequence of said gene implicated in antimicrobial defence, and
b) measuring the level of expression of the reporter gene.

2. The method according to claim 1, wherein the reporter gene is the GFP (Green Fluorescent Protein) gene, preferably the eGFP (enhanced Green Fluorescent Protein) gene.

3. The method according to claim 1 or 2, wherein the promoter sequence is selected from the group comprising DEFB4 promoter sequence, DEFB103 promoter sequence, CAMP promoter sequence, CCL20 promoter sequence and CCR6 promoter sequence.

4. The method according to claim 3, wherein the promoter sequence is selected from the group comprising human DEFB4 promoter sequence (SEQ ID NO: 1), human DEFB103 promoter sequence (SEQ ID NO: 2), human CAMP promoter sequence (SEQ ID NO: 3), human CCL20 promoter sequence (SEQ ID NO: 4) and human CCR6 promoter sequence.

5. The method according to any of the claims 1 to 4, wherein said method further comprises a step c) of comparing the level of expression of the reporter gene as measured in step b) with the level of expression of the reporter gene in the absence of said test compound.

6. The method according to any of the claims 1 to 5, wherein the host cell is infected by *Shigella,* preferably *Shigella flexneri.*

7. The method according to any of the claims 1 to 6, wherein the host cell has been transformed with a vector selected in the group consiting of plasmids pLenti/R4R2/V5DEST-pBS5 comprising human DEFB4 promoter fused to the eGFP reporter nucleic acid sequence, pLenti/R4R2/V5DEST-pBS7 comprising human DEFB103 promoter fused to the eGFP reporter nucleic acid sequence, and pLenti/R4R2/V5DEST-pBS9 comprising human CAMP(LL37) promoter fused to the eGFP reporter nucleic acid sequence, which plasmids are present in the Stb13 *E. coli* bacterial strains deposited at the CNCM on April 4, 2008 with the numbers I-3965, I-3966 and I-3967 respectively.

8. The method according to any of the claims 1 to 6, wherein the host cell is an epithelial cell selected from the group comprising human colonic epithelial cells HT29 (ATCC HTB-38D), Caco-2 (ATCC HTB-37) or TC7 cell line which is a subclone of Caco-2..

9. The method according to any one of claims 1 to 8, wherein said method permits to select compounds which can be useful for treating and/or preventing a microbial infection, preferably a *Shigella* infection, and more preferably a *Shigella flexneri* infection.

10. Pharmaceutical composition comprising at least one compound identified by the method according to any of the claims 1 to 9 and, optionally, a pharmaceutically acceptable carrier.

11. Use of at least one compound identified by the method according to any of the claims 1 to 9 for preventing and/or treating an microbial infection or for preventing and/or treating cancer in a subject.

12. The use according to claim 11 for preventing and/or treating a *Shigella* infection in a subject, preferably a *Shigella flexneri* infection.
